# EUROPEAN PATENT APPLICATION

(11) **EP 3 466 484 A1**
(43) Date of publication of application: **10.04.2019**
(21) Application number: 18187987.5
(22) Date of filing: 08.08.2018
(51) Int. Cl.: A61N 1/372, A61N 1/36, A61N 1/362, G06F 21/62, H04L 9/08, H04L 9/32, H04L 29/06

(54) **METHOD FOR ENABLING A PATIENT TO GRANT ACCESS TO THEIR ELECTRONIC IMPLANT BY A TRUSTED CLINICIAN**

(30) Priority: 19.09.2017 US 201762560222 P
(71) Applicant: BIOTRONIK SE & Co. KG, 12359 Berlin (DE)
(72) Inventor: Grosskopf, Rainer Jörg, Portland, OR 97239 (US); Reinert, Michael, Tigard, OR 97223 (US); Lohmann, William Carl, Portland, OR 97229 (US)
(74) Representative: Keck, Hans-Georg

(57) **Abstract**

The present invention relates to a method for secure communication with a medical device (10), wherein a key for establishing a secure communication channel (2) between an external device (CP) and the medical device (10) is generated by an auxiliary device (PR), and wherein said generated key is retrieved by the external device (CP) from said auxiliary device (PR), wherein said secure communication channel (2) is then established between the medical device (10) and the external device (CP) using said key retrieved from the auxiliary device (PR).

## Description

Medical devices like spinal cord stimulators (SCS) provide electrotherapy to reduce pain in patients. SCS systems typically include an auxiliary device (e.g. a patient remote control device) assigned to the patient to control the therapy, and another external device (also denoted as clinician programmer or programming device) assigned to a clinician to e.g. control the therapy.

Traditional communication links between the programming device and the (implantable) medical device use a wireless link that reaches 5 cm to 10 cm (e.g. near-field communication). In these traditional systems the patient implicitly consents to the programming device-to-medical implant communication link by allowing the programming device's communication coil to be placed over the medical device/implant.

Newer implantable designs intentionally replace the near-field communication with far-field RF, e.g. Bluetooth or MICS band RF. This makes the system easier to use, but it opens it up for abuse from malicious communication attempts.

Furthermore, US 7,831,828 B2 discloses an authenticated data communication between an implantable device and an external device, particularly using authentication via a physical token. A similar procedure is disclosed in US 2012/0330380 A1.

Based on the above, it is an objective of the present invention to provide the patient with an easy and secure method for granting/revoking access to his/her medical device / implant.

This problem is solved by a method having the features of claim 1 as well as by a system having the features of claim 10. Particular embodiments of these aspects of the present invention are stated in the corresponding sub claims and/or are described below.

According to claim 1, a method for secure communication with between an external device and a medical device is disclosed, wherein a key for establishing a secure communication between the external device and the medical device is generated by an auxiliary device, and wherein said generated key is retrieved by an external device from said auxiliary device, wherein a secure communication channel is then established between the medical device and the external device using said key retrieved from the auxiliary device.

In other words, particularly, the invention considers a patient who has an electronic medical implant. The invention allows the patient to grant/revoke implant access to another person (typically a clinician). In this invention the patient's implant remote control device displays an optical code / key, this code / key is consumed by the clinician's implant programming device for the purpose of transferring control over the implant to the clinician.

Particularly, according to the present invention, a communication is deemed secure in case a key generated by the auxiliary device (operated by the patient) and retrieved by the external device (operated by the clinician) is used to establish the communication between the external device and the medical device. Thus, access is granted to the clinician via a key sent by the patient.

Particularly, the present invention introduces a more secure method for transferring control over the implant to a trusted clinician. It also avoids potential unintentional/malicious access to the implant, where the untrusted user is within RF range, but not visible to the patient. E.g. in an adjacent room.

Particularly, according to an embodiment of the method according to the present invention, the medical device is an implantable medical device that has been implanted into the patient before. Particularly, implanting the medical device is explicitly considered to not form a part of the method according to the present invention.
Furthermore, according to an embodiment of the method according to the present invention, the auxiliary device is a patient remote control device that is adapted to receive an input from the patient (or another authorized user) and to control the medical device based on said input. Particularly, the auxiliary device comprises a display for graphically displaying information (e.g. said key).

Furthermore, according to an embodiment of the method according to the present invention, the external device is a physician/clinician programming device that is adapted for controlling the medical device via said secure communication channel.

Furthermore, according to an embodiment of the method according to the present invention, retrieving the key from the auxiliary device comprises the steps of displaying said key as a graphic code (image ID) by the auxiliary device (e.g. via an optical display of the auxiliary device) and optically detecting (e.g. scanning) the key / code by the external device (using e.g. an image sensor / camera of the external device).

Furthermore, according to an embodiment of the method according to the present invention, retrieving the key from the auxiliary device comprises the steps of transmitting the key from the auxiliary device to a web site in a network (particularly the internet), wherein the key is then displayed as a graphic code (image ID) on said web site and optically detected (e.g. scanned) by the external device (using e.g. an image sensor / camera of the external device).

Furthermore, according to an embodiment of the method according to the present invention, the key / graphic code further comprises at least one of: a time information for limiting communication between the external device and the medical device via said channel to a pre-defined period of time, a location information for limiting the communication between the external device and the medical device to a geographic area, an identity information identifying a person (e.g. clinician) for limiting communication between the external device and the medical device to a certain person (e.g. clinician).

Furthermore, according to an embodiment of the method according to the present invention, the external device is always allowed to read information from the medical device, wherein a key / graphic code is merely generated by the auxiliary device and retrieved by the external device in case parameters of the medical device are to be changed via the external device (e.g. change of therapy by the clinician).

Furthermore, according to an embodiment of the method according to the present invention, a key is only generated and/or displayed by the auxiliary device (or on said web site) when the patient places a permanent magnet in the vicinity of the medical implant in addition.

Furthermore, according to an embodiment of the method according to the present invention, the medical device is one of: a device for neurostimulation, particularly for spinal cord stimulation (SCS), a cardiac pacemaker.

Yet another aspect of the present invention relates to a system for secure communication between an (e.g. implanted) medical device and an external device, comprising an (e.g. implanted) medical device, an auxiliary device and an external device, wherein for establishing a secure communication between the medical device and the external device, the auxiliary device is configured to generate a key, and wherein said external device is configured to retrieve said generated key from said auxiliary device, wherein the medical device and the external device are configured to establish a secure communication channel between the medical device and the external device using said key retrieved from the auxiliary device.

Furthermore, according to an embodiment of the system according to the present invention, said auxiliary device is a patient remote control device that is adapted to receive an input from the patient (or another authorized user) and to control the medical device based on said input. Further, particularly, the auxiliary device comprises a display for graphically displaying information such as the key / graphic code.

Furthermore, according to an embodiment of the system according to the present invention, the external device is a physician programming device that is adapted for controlling the medical device via said secure communication channel.

Furthermore, according to an embodiment of the system according to the present invention, the auxiliary device is configured to generate said key and to display said key as a graphic code (e.g. via an optical display of the auxiliary device), wherein the external device is configured to optically detect (e.g. scan) the key / graphic code (using e.g. an image sensor / camera of the external device).

Furthermore, according to an embodiment of the system according to the present invention, the auxiliary device is configured to generate said key and to transmit the key to a web site in a network (particularly the internet) for displaying said key on said web site as a graphic code, and wherein the external device is configured to optically detect (e.g. scan) the key from the web site (using e.g an image sensor / camera of the external device).

Furthermore, according to an embodiment of the system according to the present invention, the graphic code can be a barcode or a QR-code or another graphic code.

Furthermore, according to an embodiment of the system according to the present invention, the auxiliary device is configured to generate the key/graphic code such that the key/graphic code further comprises at least one of: a time information for limiting communication between the external device and the medical device via said channel to a pre-defined period of time, a location information for limiting the communication between the external device and the medical device to a geographic area, an identity information identifying a person (e.g. clinician) for limiting communication between the external device and the medical device to a certain person (e.g. clinician).

Furthermore, according to an embodiment of the system according to the present invention, the system is configured to always allow the external device to always read information from the medical device without requiring a key, and such that a key is merely generated by the auxiliary device and retrieved by the external device in case at least one parameter of the medical device is to be changed via the external device (e.g. change of therapy by the clinician).

Furthermore, according to an embodiment of the system according to the present invention, the auxiliary device may be configured to only generate a key and/or display the key via the auxiliary device (or via said web site) when a permanent magnet has been placed in the vicinity of the medical implant in addition.

Furthermore, according to an embodiment of the system according to the present invention, the medical device is one of: a device for neurostimulation, particularly for spinal cord stimulation (SCS), a cardiac pacemaker.

Advantageously, the present invention includes explicit patient consent to transfer control over the implant. Furthermore, the method/system according to the present invention includes a visible element of security, which improves user confidence. Particularly, this improved security is due to keeping patient and clinician involved. As a consequence, the medical device comprises an improved usability and is less complicated to operate. Finally, the invention enables the use of off-the-shelf programming hardware platforms, instead of expensive traditional designs with custom hardware.

Furthermore, detailed embodiments and features of the present invention will be described below with reference to the Figure, wherein
- Fig: 1: shows a schematical representation of a method/system according to the present invention.

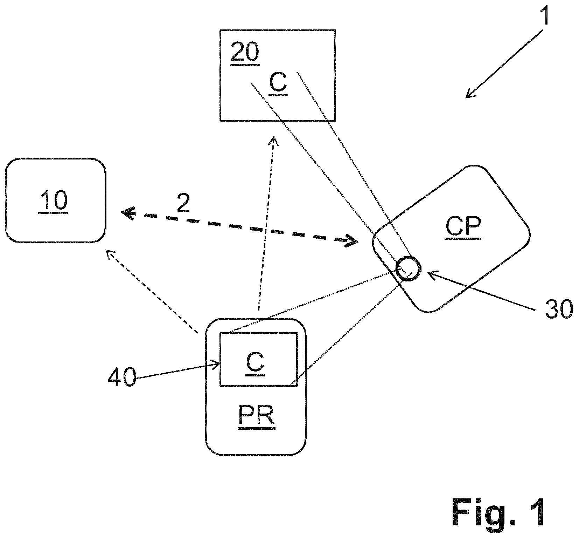

Figure 1 illustrates an example of a method / system 1 for spinal cord stimulation (SCS). Such a system comprises an implantable medical device 10 for delivering SCS that comprise a first and a second implantable percutaneous lead (not shown) that are implanted into a targeted location in the epidural space. Such leads may be replaced by a paddle lead or other type of SCS leads. Particularly, electrical stimulation pulses are delivered via said leads that comprise a plurality of electrodes. It is however to be noted that the invention at hand can also be applied to other medical devices.

To control such an implantable medical device 10 and to grant other devices access to the medical implant 10, the system 1 comprises an auxiliary device PR, here in form of a patient remote control device PR. Particularly, a display 40 of the remote control device PR is used to display an authorization key in the form of a graphic code C (e.g. barcode, QR-code etc.). The external device CP that shall have access and can be formed by a programming device that is operated by a clinician in order to control the medical device 10 (i.e. change parameters, alter therapy etc.) can then scan the code C using an image sensor 30. The image sensor 30 can be an integral part of the external device CP. However the external device CP may also consist of separate devices that are linked to exchange information (e.g. an image sensor / camera 30 for scanning the code C, which image sensor / camera 30 that is connected to a separate device of the external device for actually programming/controlling the medical device 10). The information encoded in the image / graphic code C contains the (authorization) key which enables the external device CP to communicate with the implant 10. Particularly, this image scanning process introduces the essential element of proximity and consent between physician and patient.

The information encoded in the image / code C may include, but is not limited to: said authorization key (or several such keys), which is/are designed to authenticate the far-field RF link between the external device CP and the implant 10, an element of time, which one can use to limit the authorization to a period of time, an element of location, which one can use to limit the authorization to a geographic area, an identity of a clinician who the patient intends to authorize.

Additionally, the system 1 could include a web site 20 where the patient logs in, and where the patient could display a graphic code C to authorize a clinician access. The clinician's external device CP would then scan the code C from the patient portal webpage 20. This would solve the issue of a patient forgetting his/her auxiliary device PR when visiting the clinic. This web-based mechanism requires the auxiliary device PR to be in network contact with the web portal system sometime prior to the clinic visit.

Additionally, the present invention can be used to control different levels of access. E.g. the implant 10 could allow a clinician programmer to read information, but requires the optical key exchange described in this invention before allowing therapy changes.

Additionally, this invention can be used in conjunction with other methods that establish proximity. E.g. the user places a permanent magnet over the implant 10 and also performs the optical key exchange described herein.

## Claims

1. A method for secure communication with a medical device (10), wherein a key for establishing a secure communication channel (2) between an external device (CP) and the medical device (10) is generated by an auxiliary device (PR), and wherein said generated key is retrieved by the external device (CP) from said auxiliary device (PR), wherein said secure communication channel (2) is then established between the medical device (10) and the external device (CP) using said key retrieved from the auxiliary device (PR).

2. The method according to claim 1, **wherein** the medical device (10) is an implanted medical device (10).

3. The method according to claim 1 or 2, **wherein** the auxiliary device (PR) is a remote control device that is adapted to receive an input from a patient to which the medical device (10 is associated and to control the medical device (10) based on said input.

4. The method according to one of the preceding claims, **wherein** the external device (CP) is a programming device that is adapted for controlling the medical device (10) via said secure communication cannel (2).

5. The method according to one of the preceding claims, **wherein** retrieving the key from the auxiliary device (PR) comprises the steps of displaying said key as a graphic code (C) by the auxiliary device (PR) and optically detecting the graphic code (C) by an image sensor (30) the external device (CP).

6. The method according to one of the claims 1 to 4, **wherein** retrieving the key from the auxiliary device (PR) comprises the steps of transmitting the key to a web site (20) in a network, wherein the key is then displayed as a graphic code (C) on said web site (20) and optically detected by an image sensor (30) of the external device (CP).

7. The method according to one of the claims 5 or 6, **wherein** the graphic code (C) is one of: a bar code, a QR-code.

8. The method according to one of the claims 5 to 7, **wherein** the graphic code (C) further comprises at least one of: a time information for limiting communication between the external device (CP) and the medical device (10) via said channel (2) to a pre-defined period of time, a location information for limiting the communication between the external device (CP) and the medical device (10) to a geographic area, an identity information identifying a person for limiting communication between the external device (CP) and the medical device (10) to said person.

9. The method according to one of the preceding claims, **wherein** the medical device (10) is one of: a device for neurostimulation, particularly for spinal cord stimulation (SCS), a cardiac pacemaker.

10. A system for secure communication between a medical device (10) and an external device (CP), comprising a medical device (10), an auxiliary device (PR) and an external device (CP), wherein for establishing a secure communication channel (2) between the medical device (10) and the external device (CP), the auxiliary device (PR) is configured to generate a key, and wherein said external device (CP) is configured to retrieve said generated key from said auxiliary device (PR), wherein the medical device (10) and the external device (CP) are configured to establish said secure communication channel (2) between the medical device (10) and the external device (CP) using said key retrieved from the auxiliary device (PR).

11. The system according to claim 10, **wherein** the auxiliary device (PR) is a remote control device (CP) that is adapted to receive an input from a patient to which the medical device is associated and to control the medical device (10) based on said input.

12. The System according to claim 10 or 11, **wherein** the external device (CP) is a programming device (CP) that is adapted for controlling the medical device (10) via said secure communication channel (2).

13. The system according to one of the claims 10 to 12, **wherein** the auxiliary device (PR) is configured to generate said key and to display said key as a graphic code (C) via an optical display (40) of the auxiliary device (PR), wherein the external device (CP) is configured to optically detect the graphic code (C) using an image sensor (30).

14. The system according to one of the claims 10 to 12, **wherein** the auxiliary device (PR) is configured to generate said key and to transmit the key to a web site (20) in a network for graphically displaying said key on said web site (20) as a graphic code (C), and wherein the external device (CP) is configured to optically detect the graphic code (C) from the web site (20) using an image sensor (30).

15. System according to one of the claim 10 to 14, **wherein** said graphic code (C) further comprises at least one of: a time information for limiting communication between the external device (CP) and the medical device (10) via said channel (2) to a pre-defined period of time, a location information for limiting the communication between the external device (CP) and the medical device (10) to a geographic area, an identity information identifying a person for limiting communication between the external device (CP) and the medical device (10) to said person.
